# EUROPÄISCHE PATENTSCHRIFT

(11) **EP 1 522 281 B1**
(45) Veröffentlichungstag und Bekanntmachung des Hinweises auf die Patenterteilung: **25.06.2008**
(21) Anmeldenummer: 04025267.8
(22) Anmeldetag: 29.12.1998
(51) Int. Cl.: A61F 2/34, B23G 1/00

(54) **Hüftgelenkpfanne**
Acetabular cup
Cupule acétabulaire

(30) Priorität: 29.12.1997 DE 19757799
(43) Veröffentlichungstag der Anmeldung: 13.04.2005
(62) Teilanmeldung aus: 98966679.7
(73) Patentinhaber: Hörmansdörfer, Gerd, D-31303 Burgdorf (DE)
(72) Erfinder: Hörmansdörfer, Gerd, D-31303 Burgdorf (DE)
(74) Vertreter: Siekmann, Gunnar

(56) Entgegenhaltungen:
- EP-A- 0 480 551
- WO-A-97/39702
- US-A- 4 919 677

## Beschreibung

Die Erfindung betrifft eine einschraubbare künstliche Hüftgelenkpfanne. Ein spezielles Verfahren zur drehtechnischen Bearbeitung von Werkstücken und eine bevorzugte Anwendung des Verfahrens werden ebenfalls beschrieben.

Im Prinzip ist die konventionelle Drehtechnik ein seit sehr langer Zeit bekanntes Verfahren für die spanende Herstellung von Werkstücken z.B. aus Holz, Metall oder Kunststoff. In jüngerer Zeit hat die Drehtechnologie durch die Einführung und Fortentwicklung numerischer Steuerungen eine rasante Ausweitung ihrer Möglichkeiten erfahren. So ist heute z.B. die Einhaltung einer konstanten Schnittgeschwindigkeit entlang der Oberflächenkontur überhaupt kein Problem. Selbst komplizierteste Geometrien sind mittels einer entsprechenden Programmierung relativ einfach zu verwirklichen und in sehr kurzen Bearbeitungszeiten herstellbar. Daneben wurden derartige Maschinen durch die Ausrüstung mit Werkzeugantrieb weiter aufgewertet, weil so komplizierte Werkstücke in einer Aufspannung dreh- und frästechnisch fertig bearbeitet werden können. Trotzdem bestehen hier gewisse Einschränkungen, welche entweder den Faktor Zeit oder bestimmte geometrische Gestaltungen betreffen. Es ist z.B. eine Tatsache, daß die drehtechnische Herstellung generell deutlich kürzere Bearbeitungszeiten ermöglicht als das Fräsen. Außerdem ergeben sich beim Drehen bessere Oberflächenqualitäten. Wenn wegen der Werkstückgeometrie nur eine frästechnische Herstellung in Frage kommt, muß daher zwangsläufig eine deutlich längere Bearbeitungszeit bzw eine ungleichmäßigere Oberfläche in Kauf genommen werden. Aber auch mit einer frästechnischen Herstellung sind die geometrischen Möglichkeiten limitiert. So kann beispielsweise jede Ecke einer gefrästen Kontur in der Radialebene der Fräserachse niemals scharfkantiger sein als der Radius des verwendeten Fräsers. Scharfkantige Konturen sind zwar mittels Räumen, Stoßen oder durch Erodieren erzielbar, jedoch muß dazu das Werkstück auf eine andere Maschine übernommen werden. Im Falle des Erodierens ist der Zeitbedarf extrem hoch. Zwar sind für die spanende Herstellung unrunder Konturen seit einigen Jahren sogenannte Formbohr- bzw. Formdrehgeräte auf dem Markt, jedoch haben diese Geräte ihren Preis und erfordern so eine kapitalmäßige Investition entsprechender Größenordnung. Außerdem sind sie nur an die vorgesehene Schnittstelle anschließbar und auf eine vorgegebene Kontur mit zweidimensionaler Unrundheit beschränkt.

Bereits früher hatte es Versuche gegeben, Drehmaschinen durch Anbau spezieller mechanischer Baugruppen für die Bearbeitung von unrunden Werkstücken zu ertüchtigen. Eine entsprechende Maschine wird in der Deutschen Offenlegungsschrift DE 25 15 106 vorgeschlagen. Neben dem sehr aufwendigen und anfälligen Bauaufwand krankt diese Maschine an der extremen Limitierung ihrer Möglichkeiten, welche sich ohnehin nur auf die Erzeugung einer zweidimensionalen Unrundgeometrie beschränken.

Die geometrischen Möglichkeiten der Unrundbearbeitung sind bezüglich auf eine Drehmaschine aufrüstbarer Werkzeuge z.B. erweiterbar, wenn der Schneidenantrieb frei programmierbar angesteuert werden kann. Ein solches Werkzeug ist z.B. aus der Deutschen Offenlegungsschrift DE 35 09 240 A1 bekannt. Hier werden piezoelektrische oder magnetostriktive Stellglieder herangezogen, um eine dynamische Schneidenverschiebung relativ zum Werkstück mittels einer entsprechenden elektrischen Ansteuerung zu verwirklichen. Hiermit sind jedoch nur sehr kleine Stellwege erzielbar. Es wäre zwar technisch möglich, z.B. durch Anwendung eines magnetodynamischen Systems zu wesentlich größeren Stellwegen zu gelangen, doch wären diese wie zuvor auf eine einzige Bewegungsachse limitiert. Zur Erzielung bestimmter dreidimensionaler Unstetbearbeitungen wäre es notwendig, durch Hinzufügen einer zweiten oder sogar dritten jeweils orthogonal angeordneten Bewegungseinheit ein Werkzeug mit komplexen Bewegungsrichtungen zu schaffen, allerdings wäre dieses aufwendig im Bau und anspruchsvoll in Bezug auf die erforderliche Ansteuerelektronik. Ein derartiges Werkzeug ist bisher nicht verfügbar.

Es sind auch spezielle Drehmaschinen bekannt, welche für die Unrundbearbeitung z.B. von Kolben für Verbrennungsmotoren entwickelt worden sind. Moderne Kolben besitzen nämlich einen leicht unrunden, in der Regel elliptischen Querschnitt, um die anisotrope Ausdehnung bei Erwärmung zu kompensieren. Allerdings besteht hier nur eine sehr geringe Abweichung von der Kreisform, wobei die Kontur außerdem einen weich fließenden Verlauf aufweist. Sprünge oder extreme Unstetigkeiten sind dort nicht vorhanden. Demgemäß besteht hinsichtlich der baulichen Auslegung einer derartigen Maschine kein sehr hoher Schwierigkeitsgrad. Es genügt im Prinzip, den Drehmeißel in der den Durchmesser betreffenden x-Achse mit geringer Amplitude schwingen zu lassen, während der Schlitten entlang dem Werkstück in der z-Achse verfahren wird. Dabei wird die Auslenkungskurve der Drehmeißelspitze einen mehr oder weniger sinusförmigen Verlauf zeigen, sodaß extreme Beschleunigungen gar nicht erforderlich sind. Diese wären trotz der reduzierten Masse des Systems ohnehin nur schwer zu realisieren. Es versteht sich, daß derartige Maschinen eine Verkopplung der Werkstückrotation zur Bewegung der x-Achse erfordern, jedoch ist der Vorschub in der z-Achse frei gestaltbar. Tatsächlich ist die Erzeugung der unrunden Kontur dabei auf die zweidimensionale Durchmesserebene beschränkt und wird mittels der z-Achse auf die dritte Dimension lediglich ausgedehnt. Die z-Achse ist dabei jedoch in die unrunde Konturerzeugung nicht wirklich einbezogen. Ein Verfahren des Schlittens entlang der z-Achse in Sprüngen oder mit einer etwa überlagerten Oszillation ist nicht vorgesehen.

Eine Sondermaschine der oben genannten Art wird z.B. in der Deutschen Offenlegungsschrift DE 40 31 079 A1 beschrieben, wobei vorgeschlagen wird, für die Ansteuerung des für die oszillierende Bewegung des Drehmeißels vorgesehenen Antriebs (z.B. elektrischer Linearmotor oder hydraulisches System) neben der vorhandenen Maschinensteuerung eine zusätzliche Rechnersteuerung z.B. in Gestalt eines Personalcomputers heranzuziehen. Ohne Abänderung des zugrunde liegenden kinematischen Verfahrens ist jedoch eine derartige Maschine in ihren Möglichkeiten auf die vorgesehene und ähnliche Anwendungen beschränkt. Außerdem ist eine solche Sondermaschine in der Anschaffung verhältnismäßig teuer.

Es bestand daher die Aufgabe zur Schaffung eines Verfahrens für die drehtechnische Bearbeitung von Werkstücken mit Unregelmäßigkeiten oder Unstetigkeiten der Kontur, welches einerseits die an der Maschine bestehenden Gegebenheiten hinsichtlich des Kreuzschlittens und der NC-Steuerung nutzen, ohne Zusatzgerätschaften auskommen, die mit der Massenträgheit einhergehenden Plobleme überwinden und gleichzeitig den Freiheitsgrad bezüglich der Unstetigkeit der erzeugbaren Kontur um wenigstens eine zusätzliche Dimension erweitern sollte. Dabei war auch angestrebt, bisherige frästechnische Operationen durch das neue Verfahren so weit wie möglich abzulösen.

Die genannte Aufgabe wird durch ein als Humpeldrehen bezeichnetes drehtechnisches Verfahren gelöst, wobei das Werkstück im Futter der Maschinenspindel mit einer - vorzugsweise konstanten - Drehzahl rotiert, und dabei der Kreuzschlitten mit dem Zerspanungswerkzeug unter Benutzung einer Gewindeprogrammierung in der Steigungsachse synchronisiert zum Spindelwinkel verfahren wird und bestimmte unrunde, aus geometrischen Übergangselementen zusammengesetzte Konturen mittels einer Programmierung aus Sprungfunktionen durch Verknüpfung von Befehlssätzen mit Werten für die Adreßparameter X (Durchmesser), Z (Länge) und F (Steigung) erzeugt werden, wobei mindestens für einen dieser Parameter in der Programmsatzkette eine Folge aus humpelnden Wertegruppen mit mindestens einem Zahlenwert in jeder Wertegruppe verwendet wird. Das Verfahren ist durch Heranziehung des Parameters Y (Höhe) bei entsprechend ausgerüsteten Maschinen erweiterbar.

Die bei den meisten Bearbeitungsaufgaben in der Programmsatzkette für mindestens einen Adreßparameter zwischen den Zahlenwerten gebildeten Inkremente stellen eine humpelnde Folge aus Wertegruppen mit mindestens einem Zahlenwert in jeder Wertegruppe dar, wobei z.B. die entsprechenden Zahlenwerte innerhalb der einen Wertegruppe größer sind als die innerhalb der anderen und/oder das Vorzeichen innerhalb der einen Wertegruppe positiv und innerhalb der anderen Wertegruppe negativ ist. Im Prinzip bilden die für einen bestimmten Adreßparameter programmierten Werte in der Programmsatzkette eine Folge von Zahlenwerten, in welcher die befohlenen Sprungfunktionen als sogenannte Humpelschritte zum Ausdruck kommen.

Besondere Bedeutung erlangt das Verfahren durch seine Anwendungsmöglichkeit in allen drei Dimensionen, selbst ohne Heranziehung der y-Achse. Diese bearbeitungsmäßige Freiheit ist darauf zurückzuführen, daß Humpeischritte mittels X, Z und F jeweils allein oder in Kombination miteinander programmierbar sind.

Das Verfahren wird durch ein Sprungsystem erweitert, wobei die herzustellenden Unstetigkeiten in aufeinander folgenden Sequenzen aus geometrisch gegeneinander versetzten Drehzyklen erzeugt werden.

Das Verfahren benötigt weder Speziaigerätschaften noch zusätzliche NC-Steuerungen und beruht allein auf der Anwendung der mit der Maschinensteuerung und der entsprechenden Software gegebenen Möglichkeiten und wird lediglich begrenzt durch die Dynamik des Gesamtsystems. Hierzu sind z.B. die bekannten Befehlssätze G 31, G 33, G 34, G 37 bzw. G 131 usw., sowie z.B. die Adreßparameter X (Durchmessermaß), Z (Längenmaß), F (Gewindesteigung), B (Anlauflänge), P (Überlauflänge), C (Startwinkel der Spindel), H (Bezugsrichtung für F) und E (Steigungsänderung) verwendbar oder einfügbare Blöcke mit individueller Software. Es ist auch nicht ausgeschlossen, daß aufgrund des hier vorgeschlagenen Verfahrens in der Zukunft erweiterte Programmierungsmöglichkeiten seitens der Industrie serienmäßig angeboten werden.

Die oben angesprochene Dynamik des Gesamtsystems setzt sich zusammen aus der mechanischen und elektronischen Dynamik der Maschine. Dabei ist die mechanische Dynamik abhängig von der Masse des Kreuzschlittens und der Reaktionsgeschwindigkeit des Antriebs z.B. aus Gewindespindeln, Motoren und Getriebe. Demgegenüber ist die elektronische Dynamik durch die Rechengeschwindigkeit der Steuerung und deren Verknüpfung mit den elektromotorischen Antrieben vorgegeben. Demgemäß sind Drehmaschinen der allerneuesten Generation mit digitalen Antrieben und schnellsten Rechnern für extreme Unstetbearbeitungen geeignet, während die Anwendung des Verfahrens auf älteren Maschinen entsprechend eingeschränkt ist. Diese Beschränkung kann teilweise durch die Benutzung reduzierter Schnittgeschwindigkeiten während der Zerspanung zurückgedrängt werden, weil sich daraus niedrigere Spindeldrehzahlen und entsprechend reduzierte Vorschubgeschwindigkeiten ergeben.

Eine sehr einfache Anwendung des Verfahrens besteht z.B. in der drehtechnischen Herstellung exzentrischer Zapfen. Hierzu wird mittels einer Verkettung von Befehlssätzen z.B. mit G 33 eine in Bezug auf das Werkstück drehwinkelmäßige Auflösung von 180° realisiert, indem jeweils Anfahrkoordinaten in X und Z sowie eine Steigung in F programmiert werden, wobei die zwischen den für den genannten Winkelschritt von jeweils 180° in Z programmierten Werten liegenden Inkremente im Prinzip dem halben programmierten Steigungswert entsprechen müssen. Demgegenüber springen die Werte für X bei jedem 180°-Halbschritt zwischen einem größeren und einem kleineren programmierten Durchmesserwert hin und her, wobei theoretisch der Mittelwert dem Durchmesser, und die halbe Differenz der Exzentrizität des herzustellenden Zapfens entsprechen. Zwecks Vereinfachung des Programmieraufwandes können die in Z bzw. in der Durchmesserachse sich wiederholenden Sprünge bei einigen Steuerungen als sogenannte Variable eingegeben werden. Da für das beschriebene Bearbeitungsbeispiel die Durchmesseränderung in der Regel größer als der beabsichtigte Vorschub in Gestalt der Steigung ist, wird die Maschinensteuerung im Normalfall die programmierte Steigung mit dem Vorschub der X-Achse verrechnen. Daher muß für die Steigung unter F der bezüglich des Durchmessers pro Umdrehung programmierte Weg, also die doppelte Durchmesserdifferenz, eingegeben werden, wenn nicht der Umsprung durch Befehlssätze z.B. mit H unterbunden wird. Aus der beschriebenen Programmierung ergibt sich eine theoretische Bahnkurve des Kreuzschlittens in Gestalt einer fortlaufenden Zick-Zack-Linie. Tatsächlich wird aufgrund der verschiedenen dämpfend wirkenden Faktoren, wie z.B. hoher Kreuzschlittenmasse und ungenügender Steifigkeit des Regelkreises, ein sich ständig wiederholender quasi sinusartiger Bewegungsablauf des Kreuzschlittens während des Vorschubs entlang des Werkstücks erzielt, sodaß trotz einer im Prinzip primitiven Programmierung eine erstaunliche Rundheit des exzentrischen Zapfens resultiert. Andererseits ergibt sich aus dieser Verzerrung, daß die am Werkstück nachmessbaren Abmessungen nicht exakt den programmierten Werten entsprechen. Daher müssen die zu programmierenden Zahlenwerte anhand von Probestücken ermittelt werden. Danach sind diese allerdings mit hoher Genauigkeit auf der jeweiligen Maschine reproduzierbar.

Die oben beschriebene Vorgehensweise ist für die drehtechnische Herstellung elliptischer Körper abwandelbar, indem die programmierte Zick-Zack-Kurve mit doppelter Auflösung, also mit drehwinkelmäßigen Schritten von 90°, festgelegt wird. Nun beschreiben die beiden alternierend programmierten Durchmesser den theoretischen Größf- bzw. Kleinstdurchmesser der Ellipse. Die dann gewöhnlich von der Steuerung in der X-Achse verrechnete Steigung muß dann mit der vierfachen Durchmesserdifferenz programmiert werden.

In entsprechender Weise wird vorgegangen, wenn ein Polygon (sogenanntes Gleichdick) hergestellt werden soll, wobei eine Auflösung des Winkelschritts von 60° erforderlich ist. Eine derartige Bearbeitung ist z.B. in Gestalt einer planseitig eingestochenen Nut interessant, wie sie heute z.B. als Schmiernut von Anlaufscheiben oder Reinigungsnut an Bremsscheiben bekannt ist. Bei den genannten Beispielen ist eine exakt beschriebene Nutenbahn für die ordnungsgemäße Funktion nicht erforderlich, sodaß eventuelle Bahnabweichungen bedeutungslos sind.

Bei den oben beschriebenen Beispielen handelt es sich um relativ harmonische Unrundbearbeitungen mit konstantem Vorschub in der Längsachse bei fest programmierter Steigung. Es ist ohne weiteres möglich, die beschriebene Programmierung durch Einfügen von Hilfspunkten zu erweitern und so zu einer perfektionierten Kontur zu gelangen. Das erfindungsgemäße Verfahren geht hier jedoch noch weiter, indem für die spanende Herstellung von Werkstücken mit größerer Unstetigkeit bzw. Eckigkeit der Kontur, oder der Realisierung einer höheren Bahngenquigkeit die Heranziehung von variierenden Steigungswerten - z.B. auch in Verbindung mit einer feineren Auflösung der Kontur - vorgeschlagen wird. Im Programm wird die zur Erzielung einer bestimmten Kontur vom Kreuzschlitten abzufahrende Bahn dann aus verketteten Sätzen z.B. mit G 33 beschrieben und für jeden Programmsatz eine andere Steigung festgelegt, wobei im Extremfall z.B. ein erster Programmsatz einen sehr kleinen, bzw ein nächster Programmsatz einen sehr großen Wert für F aufweist, usw., sodaß z.B. eine Abfolge aus weichen und ruckartigen Bewegungen des Kreuzschlittens entsteht. Mit diesem Verfahren sind drehtechnische Unstetbearbeitungen in einer großen Vielfalt realisierbar, z.B. auch auf den Mantelflächen gekrümmter Körper.

In gleicher Weise ist mittels des Verfahrens die in den Programmsätzen abgelegte Koordinatenkette aus jeweiligen X- und Z-Werten für sich allein, oder auch in Verbindung mit springenden F-Werten heranziehbar, um derartige unstete Konturverläufe zu verwirklichen. So ist z.B. der Vorschub einer oder beider Achsen als sogenannter Pilgerschritt programmierbar, wobei nach einer bestimmten Vorschubstrecke jeweils ein z.B. ruckartiger (kürzerer) Rücksprung folgt, dem wiederum eine z.B. größere Vorschubstrecke nachgeschaltet ist. Sinngemäß kann eine derartige Bearbeitung z.B. als das wechselweise Schneiden von verketteten Rechts- und Linksgewinden mit unter Umständen unsymmetrischer Gewindesteigung aufgefaßt werden.

Das Verfahren erlaubt auch die spanende Herstellung unstet verlaufender, aus einer geneigten oder gekrümmten Mantelfläche herausstehender Konturelemente, wobei mit der Seite des Drehmeißels im wesentlichen die Flanke des unstet verlaufenden Konturelements und mit der Spitze des Drehmeißels im wesentlichen die Mantelfläche bearbeitet wird. Hierbei wird durch entsprechende Programmierung von Start- und Zielpunkten sowie Steigung die Spitze des Drehmeißels auf einer im wesentlichen tangential zur Mantelfläche verlaufenden Bahn geführt und mit der Seite des Drehmeißels mittels einer programmierten Änderung der tangentialen Verfahrgeschwindigkeit und/oder Verfahrrichtung die Flanke des unstet verlaufenden Konturelements erzeugt.

Bei der beschriebenen Programmierung ist insbesondere darauf zu achten, daß die gewöhnlich mit dem Adreßparameter H bezeichnete Bezugsrichtung für F richtig verwendet wird. Bekanntlich wird unter H festgelegt, mit welcher Achse der Vorschub verrechnet wird, welcher der unter F programmierten Gewindesteigung entspricht. Ohne Angabe oder mit H=0 bezieht sich der Vorschub auf.die z-Achse, also im Prinzip auf Längs-, Kegel- und entsprechende verkettete Gewinde bis maximal 45° zur Z-Achse. Wird H auf 1 gesetzt, so betrifft die Vorschubverrechnung nun die x-Achse, also grundsätzlich Plan-, Kegel- und entsprechende verkettete Gewinde bis maximal 45° zur X-Achse. Daneben ist mit H=3 der Vorschub auf die Gewindebahn beziehbar. Bei verketteten Gewinden auf gekrümmten Flächen kann es leicht vorkommen, daß der Grenzwert von 45° überschritten wird und die Maschinensteuerung dann automatisch auf die andere Achsenverrechnung umspringt. Entweder muß diese dann z.B. durch Umrechnung ermittelt und im Programm bewußt verfälscht angegeben sein, oder es muß der Umsprung per Software unterbunden werden, falls die Steuerung einen entsprechenden Befehlssatz bereit hält.

Das Verfahren wird noch durch den Vorschlag erweitert, die aufgrund der limitierten Maschinendynamik bestehenden Anwendungsgrenzen dadurch zu überwinden, daß für extreme Bearbeitungsgeometrien eine Verschachtelung von Bearbeitungssequenzen herangezogen wird. Dabei handelt es sich um eine Art Sprungverfahren, welches in einem ersten Bearbeitungszyklus z.B. ein erstes Konturelement bearbeitet, dabei jedoch ein zweites ausläßt, um mit einer beruhigten Bahn wiederum ein drittes Konturelement abzufahren, usw.... Die beim ersten Bearbeitungszyklus ausgelassenen Konturelemente werden in einem zweiten Bearbeitungszyklus zerspant, wobei nunmehr die Konturelemente aus dem ersten Bearbeitungszyklus ausgelassen werden. Dieses Verfahren berücksichtigt das aus einer mit maximaler Verfahrgeschwindigkeit programmierten abrupten Bewegung resultierende Überschwingen des Gesamtsystems, welches ein in kurzer Entfernung folgendes Konturelement nicht in der gewünschten Weise abzufahren in der Lage ist. Zwecks Ausführung des Verfahrens ist damit wegen der z.B. zwei oder mehr Bearbeitungssequenzen zwar ein höherer Zeitaufwand erforderlich, jedoch ist dieser gegenüber einer frästechnischen Herstellung immer noch drastisch kürzer.

Mit dem Verfahren wird gleichzeitig eine bevorzugte Anwendung des Verfahrens vorgeschlagen. Diese Anwendung soll anhand von Ausführungsbeispielen gleichzeitig einer näheren Erläuterung des Verfahrens dienen.

Die vorgeschlagene Anwendung betrifft die Gewindeherstellung von selbstschneidend einschraubbaren Hüftgelenkpfannen, welche für die sogenannte zementfreie Implantation beim Menschen vorgesehen sind. Derartige Schraubpfannen sind in den unterschiedlichsten Ausführungen am Markt. Für eine zuverlässige und dauerhafte Integration und auch eine vorteilhafte Handhabbarkeit beim implantieren ist die Gestaltung des Gewindes von ausschlaggebender Bedeutung. Es ist mittlerweile bekannt, daß eine große Kontaktftäche des Implantats zum knöchernen Lager ohne Lastspitzen und ein zum Pfannenpol geneigtes Gewindeprofil gute Voraussetzungen für die Vermeidung von Lockerungen sind. Daneben muß eine solche Schraubpfanne eine gute Taktilianz besitzen, womit die während des Einschraubens von der Schraubpfanne vermittelte Fühlbarkeit des Aufsetzens des Schalenkörpers auf die vorbereitete knöcherne Aufnahmefläche im Acetabulum bezeichnet wird. Bei den bisherigen Schraubpfannentypen besteht hier Handlungsbedarf, weil bei Ihnen nach dem Implantieren entweder unerwünschte Freiräume zur knöchernen Grenzfläche hin bestehen, oder sie nur mit großer Kraftanstrengung eingeschraubt werden können, bzw. ihre Taktilianz unzureichend ist.

Eine Gruppe von Schraubpfannen ist mit einem sogenannten Flachgewinde versehen, bei welchem die seitlichen Flächen der Gewinderippe parallel zueinander stehen. Es ist üblich, die Gewinderippen zwecks Bildung von Schneidkanten in bestimmten Abständen durch das Einbringen von Spannuten zu unterbrechen. Bei dieser Gewindeart muß die Schneidkraft beim selbstschneidenden Einschrauben vollständig von der radial nach außen zeigenden Kopffläche der Gewinderippe bzw. den dortigen Schneidkanten erbracht werden. Ohne weitere Maßnahme beschreibt jedoch der durch die Kopffläche der einzelnen Gewindeflügel repräsentierte Kurvenzug in der axialen Aufsicht von der Polseite der Schraubpfanne her eine Spirale, deren genaue Bahn von der Formgestalt des Schalenkörpers der Schraubpfanne und der Gewindesteigung abhängt. Dadurch nimmt mit fortschreitender Windung der radiale Kurvenabstand vom polaren Mittelpunkt zu. Das Ende eines jeden Gewindeflügels steht daher radial weiter nach außen heraus als sein Anfang. Auf diese Weise entsteht beim Einschrauben einer derartigen Schraubpfanne ein Klemmeffekt, welcher lediglich durch die von der aufgerauhten Oberfläche des Implantats auf das knöcherne Material wirkenden Raspelkräfte gemildert wird. Daher sind derartige Implantate mit einem unnötig hohen Einschraubkraftbedarf behaftet.

Andererseits sind Schraubpfannen mit Flachgewinde bekannt, deren Gewindeflügel durch gruppenweises Überfräsen mit einem Freiwinkel versehen sind. Allerdings ergeben sich aus der gewählten Bearbeitungsart gerade kopfseitige Flächen, welche als Sehnen gegenüber dem von der jeweiligen Schneidkante gebildeten Schwenkkreis zurückversetzt verlaufen. Dadurch sind Schraubpfannen mit einem solchen Gewinde zwar relativ leicht einschraubbar, besitzen jedoch wegen der verkürzten Gewindezahnhöhe nur eine reduzierte Fläche zur Übertragung von Kräften. Sehr nachteilig ist insbesondere die Spaltenbildung im Bereich des Gewindezahnkopfes zwischen Implantat und Knochen, sowie die auf das knöcherne Substrat wirkende Hebelwirkung wegen der zu tief geschnittenen Zahnrillen. Daher halten auch solche Schraubpfannen einer kritischen Betrachtung aus rein medizinischer Sicht nicht stand.

Mit dem US-Patent 4,997,447 wird eine Schraubpfanne vorgeschlagen, deren Kopfflächen der einzelnen Gewindeflügel bogenförmig verlaufen, wobei ein Freiwinkel dadurch realisiert ist, daß der vom Pfannenpol ausgehende Radius dieses Bogens mit zunehmender Entfernung von der Schneidkante kleiner wird. Bei dieser Schraubpfanne dürfte ohne Einbußen ihres guten Einschraubverhaltens die zuvor beschriebene Gefahr der Spaltenbildung stark reduziert sein. Allerdings ist für ihre Fertigung ein recht hoher Zeitbedarf zu veranschlagen, da die vorgeschlagene Gestaltung das vollständige Abfahren der Zahnkopferstreckung mit einem Fräser erfordert.

Schraubpfannen der oben beschriebenen Art mit Flachgewinde konnten bislang nur einen gewissen Marktariteil erobern. Gegenwärtig scheinen Schraubpfannen mit sogenanntem Spitzgewinde weiter verbreitet zu sein. Doch auch bei dieser Gruppe besteht prinzipiell der zuvor beschriebene Problemkomplex hinsichtlich des unakzeptablen Einschraubverhaltens und der Spaltenbildung in der Kontaktzone. Die verschiedenen Versuche zur Reduzierung des Einschraubkraftbedarfs haben unter anderem dazu geführt, die eingefrästen Spannuten zu Lasten der Gewindeflügel sehr breit zu öffnen. Dadurch ging wertvolle Kontaktfläche verloren, verbunden mit der Bildung ausgedehnter Hohlräume bzw. von der Kraftübertragung ausgeschlossener ossärer Bereiche.

Bisher sind in Bezug auf Schraubpfannen mit Spitzgewinde keine Ausführungen mit einem Freiwinkel der einzelnen Gewindesegmente auf dem Markt erschienen. Dies wird vermutlich damit zusammenhängen, daß eine entsprechende Realisierung mit einem hohen Schwierigkeitsgrad behaftet ist, und die sich zunächst anbietende frästechnische Herstellung neben einer sehr aufwendigen Programmierung einen sehr hohen zeitlichen Bearbeitungsaufwand erfordert. Diese Schwierigkeiten sind darin begründet, daß bei Spitzgewinden je nach Verlauf der Spannuten mindestens eine der Seitenflächen des Gewindezahns zur Bildung einer Schneidkante herangezogen werden muß. Wenn nun hinter der Schneidkante ein Freiwinkel gebildet werden soll, so muß die entsprechende Seitenfläche des jeweiligen Gewindeflügels bis zur nächstfolgenden Spannut mit einem kongruenten Seitenwinkel hinterfräst werden. Dabei taucht das Problem auf, daß der Fräser bei gekrümmten Mantelflächen nicht gleichzeitig den Grund der Gewinderille konturtreu bearbeiten kann. Man hätte dann die Wahl, entweder entlang der Zahnflanke eine immer mehr zunehmende rillenartige Vertiefung in Kauf zu nehmen, oder ein entsprechend anwachsendes treppenstufenartiges Relikt. Dieses Überbleibsel müßte im Anschluß mittels mindestens eines weiteren Fräsganges entfernt werden.

Mit dem Verfahren ist es jetzt möglich, derartige Gewinde von Hüftgelenkpfannen drehtechnisch in kürzester Zeit und mit Perfektion herzustellen. Dabei spielt es keine Rolle, ob die Unstetbearbeitung zur Erzeugung eines bestimmten Verlaufs der einzelnen Gewindeflügel an deren pol-, äquator- oder kopfseitiger Fläche bzw. mehreren dieser Flächen erfolgen soll. Wegen der freien Programmierbarkeit der Bearbeitungsbahn ist nicht nur jedes beliebige Profil des Gewindezahns beherrschbar, sondern auch der jeweilige winkelmäßige Verlauf der erzeugten Gewinderippenabschnitte nahezu frei bestimmbar. Gleichzeitig ist die gesamte Gewindeabwicklung an den Schalenmantel des Pfannenkörpers perfekt anpaßbar. Daher kann die Erfindung für sämtliche bekannten Schalenformen wie z.B. sphärisch, asphärisch, paraphärisch, konisch-sphärisch, konisch, zylindrisch, parabolisch, toroidisch, usw. angewandt werden.

Das Verfahren ist problemlos mit anderen bekannten Verfahren zur Herstellung von Gewinden für Hüftgelenkpfannen kombinierbar, z.B. mit dem aus der Europäischen Patentschrift EP 0 480 551 bekannten Verfahren, bzw. dem mit der Deutschen Offenlegungsschrift DE 44 00 001 vorgeschlagenen Verfahren zur Herstellung eines Gewindes mit veränderlich modifizierbarem Gewindeprofil. Besonders vorteilhaft erscheint eine Kombination mit einem zum Pfannenpol überkippten Gewindezahnprofil und einer sich fließend ändernden Gewindesteigung gemäß der Internationalen Patentanmeldung WO 97/39702.

Eine Hüftgelenkpfanne gemäß dem Oberbegriff des Anspruchs 1 ist aus der EP-A-0480551 bekannt.

Es wird vorgeschlagen; bei künstlichen Hüftgelenkpfannen mit einem sich zum Kopf des Gewindezahns hin verjüngendem Zahnprofil die zwischen den Spannuten gebildeten Gewindeflügel jeweils mit sogenannten Schraubflächen herzustellen und mit ihrer jeweiligen Erstreckungsrichtung in Abhängigkeit vom Drallwinkel der Spannut zu verschwenken. Als Schraubflächen werden dabei solche Flächen verstanden, welche durch Rotation eines bestimmten Zahnprofils mit konstantem Durchmesser und einer Steigung um die Pfannenachse erzeugt sind. Bei einem z.B. trapezförmigen Zahnprofil sind folglich drei Schraubflächen gebildet, eine als kopfseitige und zwei als Seitenflächen. Dabei können diese Schraubflächen in ihrem Fußbereich entlang ihrer Erstreckung einer Verkürzung unterliegen, wenn das Zahnprofil bei bestimmten Mantelgeometrien der Schraubpfanne in die Mantelfläche hinein verläuft. Die der Schneide am Anfang des jeweiligen Gewindeflügels folgenden Flächen besitzen dann einen Neutralwinkel, also weder einen Klemm- noch einen Freiwinkel. Dadurch sind unerwünschte Klemmeffekte beseitigt und trotzdem ein allseitiger Knochenkontakt der Gewindeflügel sichergestellt. Damit die am Anfang des jeweiligen Gewindeflügels vorhandene Schneide ihre Wirkung optimal entfalten kann, muß sie gegenüber dem vorauslaufenden Gewindeflügel vorstehen. Dies wird erfindungsgemäß dadurch erreicht, daß für die Schraubflächen eines nachfolgenden Gewindeflügels ein größerer Radius herangezogen wird als für die Schraubflächen des vorauslaufenden Gewindeflügels. Zusätzlich können die einzelnen Gewindeflügel in ihrer Erstreckung in Abhängigkeit vom Drallwinkel der Spannuten relativ zueinander verschwenkt, wobei eine an den Drallwinkel sich annähernde Verschwenkungsrichtung bevorzugt ist, um einen Überstand der positiven Schneidkante zu realisieren.

Die Erfindung soll im folgenden hinsichtlich der bevorzugten Anwendung anhand der zwölf Zeichnungsfiguren näher erläutert werden. Es zeigen:
- Fig.01: Hemisphärische Schraubpfanne mit kopfseitig klemmendem Flachgewinde gemäß dem Stand der Technik
- Fig.02: Hemisphärische Schraubpfanne mit einem mit Freiwinkel versehenen Flachgewinde gemäß dem Stand der Technik
- Fig.03: Hemisphärische Schraubpfanne mit Flachgewinde aus Gewindeflügein mit kopfseitigen Schraubftöchen
- Fig.04: Erfindungsgemäße hemisphärische Schraubpfanne mit Spitzgewinde aus Gewindeflügeln mit allseitigen Schraubflächen
- Fig.05: Zwei Gewindeflügel der Schraubpfanne gemäß Fig.1
- Fig.06: Zwei Gewindeflügel der Schraubpfanne gemäß Fig.2
- Fig.07: Zwei Gewindeflügel mit Freiwinkel und bogenförmiger Kopffläche
- Fig.08: Zwei Gewindeflügel der Schraubpfanne gemäß Fig.3
- Fig.09: Zwei Gewindeflügel der Schraubpfanne gemäß Fig.4
- Fig. 10: Drei Gewindeflügel der Schraubpfanne gemäß Fig.3 und hochdynamische Werkzeugbahn
- Fig. 11: Drei Gewindeflügel der Schraubpfanne gemäß Fig.3 und Werkzeugbahn mittlerer Dynamik mit Sprungverfahren
- Fig. 12: Drei Gewindeflügel der Schraubpfanne gemäß Fig.3 und überschwingende Werkzeugbahn mit Sprungverfahren

Die Zeichnungsfigur 1 stellt die polseitige Ansicht einer hemisphärischen Schraubpfanne 1 mit Flachgewinde gemäß dem Stand der Technik anhand eines Beispiels in einer etwa 1,3-fachen Vergrößerung dar. Für das Beispiel wurde der Nenndurchmesser auf 54 mm, die mittlere Zahnhöhe auf 2,6 mm, die Steigung auf 5 mm, und der Bodenlochdurchmesser auf 22 mm festgelegt. Diese Basisabmessungen wurden für die Zeichnungsfiguren 2 bis 4 zum Zwecke der besseren Vergieichbarkeit beibehalten.

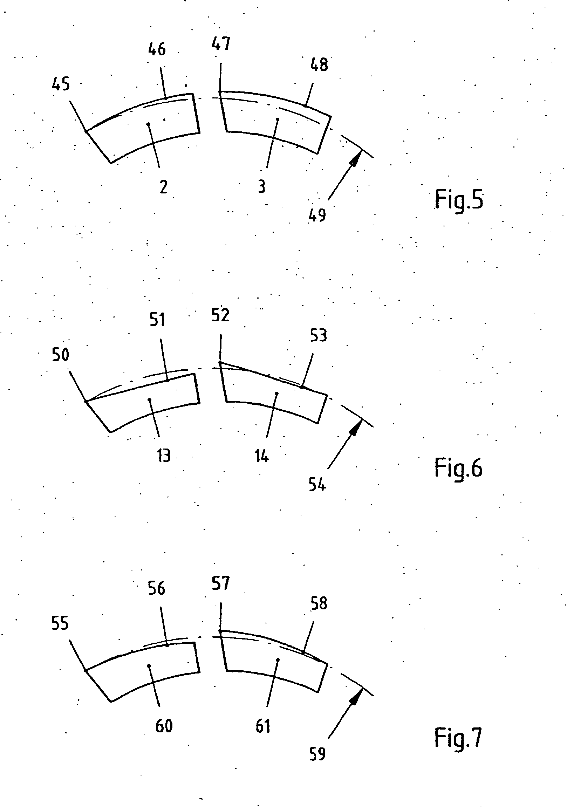

An das Bodenloch 9 der Schraubpfanne 1 schließt sich ein kuppelförmiger gewindefreier Bereich 6 des Schalenkörpers an. Der Durchmesser des Schalenkörpers wird in der Zeichnung lediglich durch den äquatorialen Randbereich 10 repräsentiert. Der Gewindezug beginnt polseitig am ersten Gewindeflügel 7 und steigt bis vor den Gewindeflügel 2 auf seine volle Höhe an. Zwei der Gewindeflügel (2,3) sind mit Kennziffern versehen, weil sie für eine Detaildarstellung in Fig.5 vorgesehen sind. Sowohl die kopfseitigen Flächen (4) als auch die jeweils am Zahnfuß zum Schalenkörper gebildeten Kanten (5) der einzelnen Gewindeflügel - mit Ausnahme des Anfangs- bzw. Endbereichs des Gewindezugs - liegen in der zweidimensionalen Darstellung jeweils auf einer spiralförmigen Kurve. Dabei umfaßt der gesamte Gewindezug etwa vier Umläufe. Der zwischen den Gewindeflügeln verlaufende Gewindegrund 8 bildet den hemisphärischen Mantel des Schalenkörpers. Zwecks Erzeugung von Spannuten (11) bzw. Schneidkanten ist die umlaufende Gewinderippe 12-fach ohne Orallwinkel geschlitzt. Dabei taucht die Schlitzung unter einem Winkel von etwa 10° ein, um am Gewindezahnkopf jeweils einen positiven Spanwinkel zu bilden.

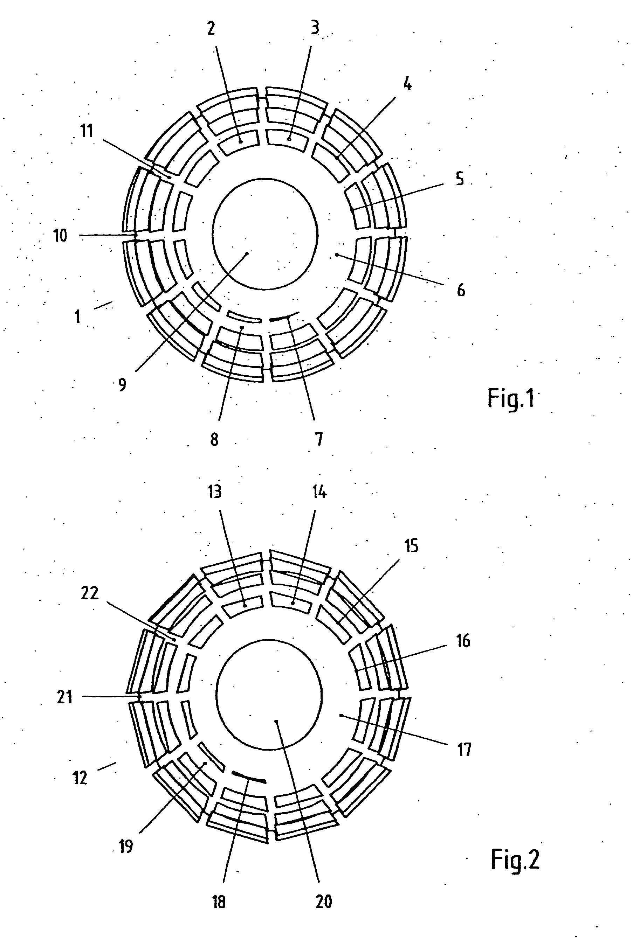

Das Ausführungsbeispiel einer Schraubpfanne 12 in Fig.2 mit Flachgewinde gemäß dem Stand der Technik ist durch eine frästechnische Nacharbeit aus der Schraubpfanne 1 hervorgegangen. Daher entsprechen Bodenloch 20, Kuppelbereich 17, Gewindegrund 19, Nenndurchmesser 21 und Schlitzung 22 ebenso wie die Kanten (16) zwischen den Gewindeflügeln und dem Schalenkörper vollständig der Fig.1. Zwecks Aufrechterhaltung einer konstanten mittleren Gewindezahnhöhe wurden wegen der hemisphärischen Schalenkontur die Gewindeflügel einzeln nachgefräst. Dabei verschob sich der polseitige Gewindeanfang auf den Gewindeflügel 18. Die geraden Außenflächen 15 der einzelnen Gewindeflügel verlaufen nun als Sehnen zum Schwenkkreis der in Einschraubrichtung jeweils vorn liegenden kopfseitigen Schneidkanten und in Synchronisation mit der Gewindeschlitzung so, daß in Bezug auf den jeweiligen Schwenkkreis Freiwinkel gebildet sind. Die Wirkung der Schneidkanten auf die Absenkung des Einschraubkraftbedarfs entfaltet sich durch den Umstand, daß der radiale Abstand der Schneidkanten von der Pfannenachse stets größer ist als der entsprechende radiale Abstand des vorauslaufenden Flügelendes. Auf zwei der mit den Kennziffern 13 und 14 versehenen Gewindeflügel soll später in der Fig.6 noch näher eingegangen werden.

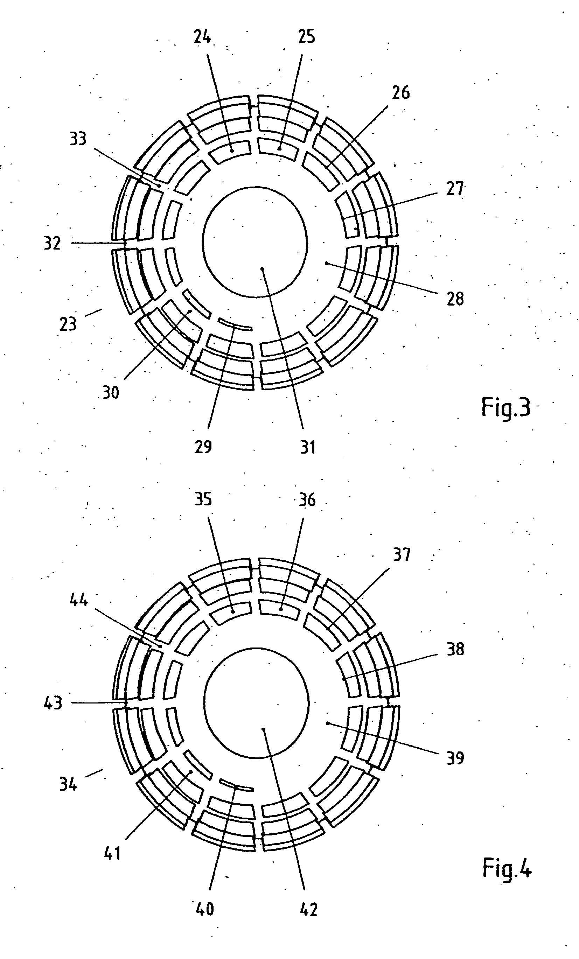

Das in Fig.3 gezeigte Ausführungsbeispiel einer Schraubpfanne 23 entspricht in seiner hemisphärischen Schalenform und seinen Grundabmessungen, sowie dem Bodenloch 31, dem sich anschließenden Kuppelbereich 28, der Kante (27) zwischen den Gewindeflügeln und dem Schalenmantel, dem Gewindegrund 30, dem Durchmesser 32 und der Gewindeschlitzung 33 wiederum dem Ausführungsbeispiel aus Fig. 1. Der Gewindezug des Flachgewindes beginnt mit einem ersten Gewindeflügel 29 geringer Zahnhöhe, dem eine Abfolge von vier weiteren Gewindeflügeln mit jeweils sprunghaft sich vergrößernder Zahnhöhe folgen, bis die Gewinderippe mit dem Gewindeflügel 24 ihre volle Höhe erreicht. Die parallel verlaufenden Flanken jedes einzelnen Gewindeflügels grenzen jeweils an einen außen liegenden. Ausschnitt aus einer zur Schraubpfannenachse koaxialen Zylinderfläche 26, wobei der zugrundeliegende Zylinderdurchmesser von Gewindeflügel zu Gewindeflügel in Stufen zunimmt. Dieses Gestaltungsprinzip ist wahlweise auch mittels eines jeweiligen Ausschnitts aus einer entsprechend koaxial liegenden Schraubfläche realisierbar. Durch die beschriebene Gestaltung ist an den Gewindeflügeln weder ein Klemm- noch ein Freiwinkel gebildet. Ein Freiwinkel ist dort überhaupt nicht erforderlich, weil die von der (z.B. durch Sandstrahlen der Schraubpfannenoberfläche erzeugten) Oberflächenrauhigkeit ausgehenden Raspelkräfte bei neutraler Relativbewegung ein Verklemmen während des Einschraubvorgangs verhindern. Damit ist zunächst die nachteilige Spaltenbildung zwischen dem Implantat und dem knöchernen Lager unterbunden. Trotzdem kommt die jeweils vorn außen liegende Schneidkante der Gewindeflügel zur Geltung, weil sie einen größeren radialen Abstand von der Pfannenachse hat als die voranlaufende Schneidkante. Das Ergebnis ist ein sehr niedriger Einschraubkraftbedarf mit überlegener Taktilianz, sowie eine herausragende Primär- und Sekundärfixation des Implantats.

Ein weiteres Ausführungsbeispiel einer erfindungsgemäßen hemisphärische Schraubpfanne 34 wird in Fig.4 vorgestellt. Auch hier wurden verschiedene Einzelheiten, namentlich das Bodenloch 42, der Kuppelbereich 39, der Gewindegrund 41, der Durchmesser 43 und die Gewindeschlitzung 44 von den zuvor gezeigten Ausführungsbeispielen unverändert übernommen. Im Gegensatz dazu handelt es sich bei dem dargestellten Gewinde um ein Spitzgewinde mit im Prinzip dreieckigem Gewindezahnprofil. Diese Tatsache ist der zweidimensionalen Darstellung nicht zu entnehmen. Ähnlich wie zuvor beginnt der Gewindezug mit einem ersten kleinen Gewindeflügel 40 und steigt in seiner Zahnhöhe in mehreren Stufen an, um vor dem Gewindeflügel 35 seine endgültige (mittlere) Zahnhöhe zu erreichen. Die vom Zahnkopf gebildete Kante (37), welche bei einem wirklich spitzen Dreiecksquerschnitt des Gewindezahns praktisch nur als Linie existiert, ist für jeden einzelnen Gewindeflügel eine Schraubenlinie mit konstantem Abstand von der Schraubpfannenachse, welche der Zeichnung nur als Bogen mit einem vom Pfannenmittelpunkft ausgehenden festen Radius zu entnehmen ist. Bei dem gewählten Spitzgewinde ist wegen des fehlenden Dralls der Spannut 44 eine Schneidkante an beiden Gewindezahnflanken gebildet. Die Schneidkante verlagert sich auf eine der Gewindezahnflanken, wenn ein entsprechender Drallwinkel der Spannut existiert. Die beidseitigen Flächen eines einzelnen Gewindeflügels des gezeigten Beispiels sind Schraubflächen, wobei die Steigung der polseitigen Fläche der Steigung der äquatorseitigen Fläche entspricht, auch wenn der optische Eindruck wegen des zum Äquator hin zunehmenden Pfannendurchmessers eine andere Situation vortäuscht. Dadurch scheint die am Zahnfuß zwischen Gewindeflügel und Schalenmantel der Schraubpfanne gebildete Kante 38 nach rückwärts in den Schalenmantel hinein zu verlaufen. Nachdem für die Schraubflächen des beim Einschrauben jeweils nächstfolgenden Gewindeflügels größere Durchmesser herangezogen werden, stehen die beidseitigen Schneidkanten gegenüber dem jeweils vorauslaufenden Gewindeflügel seitlich zum Gewindeprofil bzw. radial nach außen vor und sorgen so für einen leichtgängigen Schnitt beim Einschrauben. Auch in diesem Fall ist wegen des von den Gewindeflügeln in ihrer Erstreckung gebildeten Neutralwinkels das Auftreten von Spalten im Kontaktbereich zum Knochen unterbunden.

Die oben zum Stand der Technik und zu erfindungsgemäßen Ausführungsbeispielen gemachten Aussagen sollen im folgenden anhand vergrößert herausgezeichneter Einzelheiten besser verdeutlicht werden, weil bestimmte Details in der jeweiligen Gesamtansicht nur schwer zu erkennen sind.

In Fig.5 sind zwei Gewindeflügel 2,3 der Fig.1 vergrößert herausgezeichnet. Davon besitzt der Gewindeflügel 2 eine an der Stirn seiner kopfseitigen Fläche 46 liegende Schneidkante 45, und der Gewindefiügel 3 eine gleiche Schneidkante 47 an der entsprechenden Fläche 48. Der von der Schneidkante 45 während des Einschraubens der Schraubpfanne beschriebene Schwenkkreis 49 mit einem festen Radius um die Pfannenmittelachse ist als strich-punktierte Linie eingetragen. Es ist gut zu erkennen, daß ein Teil des jeweiligen Gewindeflügels über den Schwenkkreis herauswächst, was generell zu Klemmeffekten führen muß.

Bei der in Fig.6 gezeigten Ausführung der Gewindeflügel 13,14 gemäß dem Beispiel aus Fig.2 sind solche Klemmeffekte nicht zu befürchten, da die kopfseitigen Flächen 51 bzw. 53 nach den Schneidkanten 50 bzw. 52 mit einem Freiwinkel hinterfräst sind. Dabei wird der strich-punktierte Schwenkkreis 54 der Schneidkante 50 an keiner Stelle von der kopfseitigen Fläche des Gewindeflügels berührt. Allerdings verbleibt in diesem Bereich jeweils ein unerwünschter Freiraum. Dieser ist um so größer, je kleiner die Zahl der Spannuten ist. Hier sind insbesonders Schraubpfannen mit z.B. nur sechs Spannuten in extremer Weise gehandicapt. Die gezeigte Gestaltung wird gerne bei konischen Schraubpfannen benutzt, weil dann die Gewindeflügel sozusagen im Paket sehr rationell überfräst werden können. Aus medizinischer Sicht ist dieses Argument jedoch abzulehnen.

Der oben aufgezeigte Problempunkt ist mittels einer Gestaltung der Gewindeflügel 60,61 gemäß Fig.7 in gewissem Umfang abschwächbar. Auch hier sind die kopfseitigen Flächen 56,58 der Gewindeflügel hinter den stirnseitigen Schneidkanten 55 und 57 mit einem Freiwinkel bezüglich des Schwenkkreises 59 versehen, sodaß ein Verklemmen beim Einschrauben verhindert wird. Wegen:der Bogenform der Flächen 56, 56 ist jedoch der spaltbildende Freiraum relativ klein und daher eher akzeptabel. Allerdings erforderte diese Bogenform bisher einen hohen frästechnischen Aufwand, weil die einzelnen Gewindeflügel bei der Herstellung im Prinzip einzeln tangential abgefahren werden mußten. Mit dem erfindungsgemäßen Verfahren kann die gezeigte geometrische Gestaltung der einzelnen Gewindeflügel jetzt sehr rationell in nur einer Aufspannung auf einer CNC-Drehmaschine hergestellt werden.

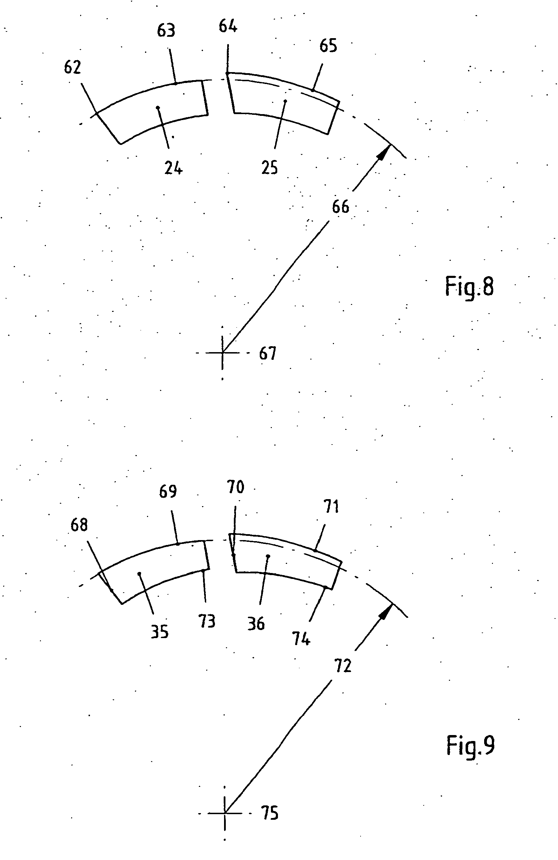

Zum Vergleich wird die mit der Erfindung insbesonders vorgeschlagene Ausführung der jeweiligen Außenflächen der einzelnen Gewindeflügel als sogenannte Schraubflächen, wie sie bereits in Fig.3 vorgestellt wurde, in Fig.8 anhand von zwei vergrößert abgebildeten Gewindeflügeln 24,25 gezeigt. Die jeweils von den Schneidkanten 62 bzw. 64 ausgehenden Kopfflächen 63 bzw. 65 der Gewindeflügel besitzen einen festen Radius, welcher jeweils ab Abstand der Schneidkante von der Schraubpfannenachse 67 definiert ist. In der Zeichnung fällt daher der durch die Schneidkante 62 verlaufende, strich-punktiert dargestellte Schwenkkreis aus dem festen Radius 66 deckungsgleich mit der Kopffläche 63 zusammen. Da der entsprechende Radius des Gewindeflügels 25 größer ist, ragt dessen Schneidkante 64 gegenüber der beim Einschrauben vorauslaufenden Schneidkante 62 des Gewindeflügels 24 vor. So kann die jeweilige Schneidkante und die sich anschließende, in einem positiven Spanwinkel angestellte Stirnfläche in das zu zerspanende Knochenmaterial eindringen und mit relativ leichtem Schnitt die Späne in die Spannut hinein abführen.

Die in Fig.9 vergrößert aus der Fig.4 herausgezeichnete Situation unterscheidet sich gegenüber der Ausführung in Fig.8 dadurch, daß das Gewinde in seinem Zahnprofil nun nicht ein Flach- sondern ein Spitzgewinde ist. Jedoch sind auch hier die äußeren Flächen der einzelnen Gewindeflügel 35,36 jeweils als Schraubflächen gestaltet. Wegen des schrägen Seitenwinkels und der Steigung bzw. Anstellung der Gewindeflügel, sowie der hemisphärischen Schalenkontur scheint die jeweils am Zahnfuß zum Schalenmatel gebildete Kante mit ihrem rückwärtigen Ende 73,74 in den Schalenkörper hinein zu verlaufen. Tatsächlich tritt jedoch bei der Rotation der Schraubpfanne kein radiales Schieben des projizierten Zahnquerschnitts auf, weil die jeweiligen Außenkanten 69,71 in ihrem Radius zur Schraubpfannenachse unveränderlich sind. Aus der Heranziehung eines dreieckigen Zahnquerschnitts für das gezeigte Beispiel ergibt sich eine Verlagerung der jeweiligen Schneidkante auf mindestens eine, bzw. für Spannuten ohne Drall, auf beide Seitenflächen des jeweiligen Gewindeflügels. In der Zeichnung ist nur die jeweils polseitige Schneidkante 68,70 zu sehen. Die jeweils rückwärtige Schneidkante ist verdeckt. Der Schwenkkreis der kopfseitigen Gewindezahnkante 69 ist mit dem festen Radius 72 um die Schraubpfannenachse 75 dargestellt. Der reduzierte Einschraubkraftbedarf dieser Ausführung ergibt sich aus dem gegenseitigen radialen Versatz der einzelnen Gewindeflügel, wodurch die einzelnen Schneidkanten gegenüber den jeweils vorauslaufenden sowohl seitlich als auch nach außen vorstehen.

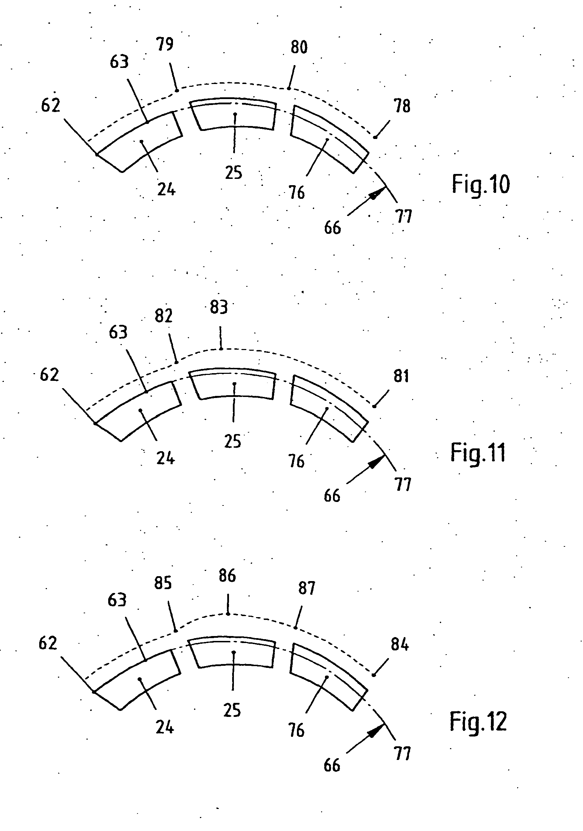

Zum besseren Verständnis der Vorgehensweise zur Ausführung des Verfahrens für die vorgeschlagene bevorzugte Anwendung zur Herstellung eines Schraubpfannengewindes werden die aus Fig.3 und 8 bekannten Gegebenheiten in den Figuren 10 bis 12 nochmals aufgegriffen. Es werden hier in jeder der Figuren die drei Gewindeflügel 24,25,76 des Flachgewindes abgebildet, sowie die Schneidkante 62 an der kopfseitigen Fläche 63 und deren strich-punktierter Schwenkkreis 77 mit dem von der Schraubpfannenachse ausgehenden Radius 66. Dabei. wurde der Abbildungsmaßstab gegenüber den vorangegangenen Figuren geringfügig verkleinert.

In der Fig. 10 ist eine von einem Bearbeitungswerkzeug (z.B. Wendeschneidplatte) beschriebene, äquidistant zu den kopfseitigen Flächen der einzelnen Gewindeflügel versetzte Bahn 78 dargestellt, welche in der gezeigten Gestalt mittels einer entsprechenden Programmierung mit einer extrem dynamischen Drehmaschine erzielbar ist. Der Abstand der Bahn von der zu zerspanenden Kontur wurde deshalb gewählt, um den Bahnverlauf in seiner vollständigen Erstreckung sichtbar zu machen. In der Bahn 78 sind zwei Unstetigkeiten 79 und 80 enthalten, welche über die Programmierung bewußt auf eine Position verlegt wurden, welche bei der nachfolgenden Bearbeitung zur Gewindeschlitzung durch Fräsen entfernt wird. Obwohl die Unstetigkeiten 79,80 der Bahn 78 Übergangsfunktionen sind, wird so zwischen den aufeinander folgenden Gewindeflügeln eine radiale Sprungfunktion bewirkt. Diese radiale Sprungfunktion besteht auf jeden Fall bezüglich der vorgeschlagenen Programmierung, wobei mindestens zwei aufeinander folgende Koordinaten gleichen Durchmessers mit einem der Bearbeitungsaufgabe angepaßten Verfahrweg in Z sowie eine entsprechende Steigung, und im Anschluß ein Durchmessersprung mit maximalem Vorschub (z.B. 100 mm/U) eingegeben werden müssen. Für ein akzeptables Bearbeitungsergebnis ist es erforderlich, daß der Übergangsbereich am Werkstück nicht breiter ist, als die vorgesehene Breite der Spannut.

Mit den meisten der heute verfügbaren CNC-Drehmaschinen ist die Erzeugung der in Fig. 10 gezeigten Schneidenbahn nicht möglich, weil ihre Gesamtdynamik nicht ausreicht, den Kreuzschlitten innerhalb der geforderten Strecke auf einen anderen Drehdurchmesser zu bewegen und dabei gleichzeitig eine ausreichende Bahngenauigkeit einzuhalten. Mit der Erfindung wird für diese Fälle ein Sprungverfahren vorgeschlagen, mit welchem dieses Problem prinzipell überwindbar ist. Der entsprechende theoretische Hintergrund soll mittels der Fig.11 verdeutlicht werden. Die anhand der Bahnkurve 81 dokumentierte Arbeitsweise sieht vor, mit einer ersten Bearbeitungssequenz lediglich den z.B. ersten, dritten, fünften, siebten usw. Gewindeflügel zu bearbeiten und dann den zweiten, vierten, sechsten usw. auszulassen. Die sich aus der Programmierung mit Sprungfunktionen aufgrund der Maschinendämpfung jeweils ergebende Übergangsfunktion der Bahn 81 muß dabei lediglich ausreichen, nach dem Punkt 82 der ersten Reaktion das Werkzeug so über die nächstfolgende Schneidkante zu heben, daß diese nicht verrundet oder beschädigt wird. Für die Rückführung des Werkzeugs auf die angestrebte Bahn steht dann z.B. bis zum Punkt 83 eine Strecke zur Verfügung, die nicht durch die Spannutenbreite limitiert ist. Es ist dann ohne weiteres möglich, in einer zweiten Arbeitssequenz die ausgelassenen Konturelemente nachzuholen und dabei die bereits bearbeiteten entsprechend zu überspringen.

Bei älteren Drehmaschinen mit entsprechender Trägheit des Regelkreises muß damit gerechnet werden, daß ein Überschwingen die Bahnkurve zusätzlich verzerrt. Dieser Effekt soll mittels der Bahn 84 in Fig. 12 verdeutlicht werden. Nach dem abrupten Reagieren der Werkzeugbewegung auf die programmierte Vorgabe bei Punkt 85 tritt ein Überschwingen der Bahn auf, welches bei Punkt 86 sein Maximum erreicht. Dieses wird im Anschluß weich auslaufend abgebaut, bis die Bahn etwa bei Punkt 87 wieder der programmierten Vorgabe entspricht. In dem Beispiel wäre der beschriebene Effekt mittels des vorgeschlagenen Sprungverfahrens in zwei Bearbeitungssequenzen gerade noch beherrschbar. Im gegebenen Fall könnte das Sprungverfahren jedoch ohne weiteres auf drei oder mehr Sequenzen ausgedehnt werden.

Das oben in verschiedenen Varianten erläuterte Verfahren ist für geschrägte Zahnkopfflächen ebenso anwendbar wie für die Seitenflächen von Gewindeflügeln z.B. gemäß Fig.9. Dabei verlagern sich die beschriebenen Sprungfunktionen entweder ganz oder teilweise von der x- auf die z-Achse. Für diese Fälle werden die vom Werkzeug beschriebenen Humpelbahnen hier zeichnerisch zwar nicht dargestellt, entsprechen jedoch vom Prinzip her denjenigen des gezeigten Sprungverfahrens für die Zahnkopfbearbeitung.

Tatsächlich sind die mit dem Verfahren gebotenen Möglichkeiten nahezu unbegrenzt. Sie ergeben sich aus der Anwendung von Gewindeschneidprogrammen und der Einbeziehung bzw. der Kombination von Humpelwerten der Adreßparameter für Durchmesser, Länge, bzw. Steigung, sowie wahlweise der Benutzung einer Pilgerschrittechnik bzw. der beschriebenen schachtelbaren Bearbeitungssequenzen. Damit sind jetzt Bearbeitungen auf CNC-Drehmaschinen sehr rationell möglich, welche zuvor zeitaufwendig und teilweise in schlechterer Oberflächenqualität durch Fräsen erzeugt werden mußten.

Die für die Anwendung des Verfahrens vorgeschlagene künstliche Hüftgelenkpfanne mit speziellem Gewinde und Gewindeflügeln aus Schraubflächen mit Neutralwinkeln hinter den Schneidkanten überzeugt durch sehr niedrige Einschraubkräfte, eine hervorragende Taktilianz und durch weitestgehend spoltenfreie Übergänge zur knöchernen Lagerfläche. Besonders vorteilhaft ist eine solche Ausführung mit Spitzgewinde, gedrallten Spannuten und relativ zueinander in Richtung zum Drallwinkel verschwenkten Gewindeflügeln. Damit ist nicht nur die Handhabung während der lmplantation deutlich verbessert, sondern auch die Primär- bzw. Sekundärfixation erheblich gesteigert und damit die Gefahr vorzeitiger Auslockerung nahezu ausgeschlossen.

## Patentansprüche

1. Einschraubbare künstliche Hüftgelenkpfanne (34) mit beliebiger äußerer Kontur des Schalenmantels, z. B. sphärisch, parasphärisch, konisch, konisch-sphärisch, parabolisch, mit einem sich zum Zahnkopf hin verjüngenden Profil des Gewindezahns, z. B. als Spitzgewinde, beliebiger Zahnstellung, z. B. neutral oder zum Pfannenpol gekippt, und beliebiger Steigung, z. B. konstanter oder variabler Steigung,
**dadurch gekennzeichnet,**
**daß** die einzelnen durch Spannuten (44) voneinander getrennten Gewindeflügel (35,36,40) oder Gewinderippen durch Schraubflächen konstanten Durchmessers gebildet sind, wobei in Einschraubrichtung nachfolgende Gewindeflügel (36) größere Durchmesser aufweisen als vorauslaufende (35).

2. Einschraubbare künstliche Hüftgelenkpfanne gemäß Anspruch 1, **dadurch gekennzeichnet, daß** die Gewindeflügel (35,36,40) in ihrer Erstreckung mit einem kleinen Winkel in Richtung zum Drallwinkel der Spannuten (44) hin relativ zueinander verschwenkt sind.

## Claims

1. A screw-in artificial acetabular cup (34) with any external contour of the shell casing, e.g., spherical, perispherical, conical, conical-spherical, parabolic, with a profile of the thread tooth tapering toward the head of the tooth, e.g., as a sharp V-thread, of any tooth position, e.g., neutral or tilted toward the socket pole, and any pitch, e.g., constant or variable pitch,
**characterized in that**
the individual thread wings (35, 36, 40) or thread ribs separated from one another by chucking grooves (44) are formed by screw surfaces of a constant diameter, whereby trailing thread wings (36) in the screw-in direction have a larger diameter than the leading thread wings (35).

2. The screw-in artificial acetabular cup according to Claim 1,
**characterized in that**
the thread wings (35, 36, 40) are swiveled relative to one another with a small angle in the direction of the angle of twist of the chucking grooves (44) in their extent.

## Revendications

1. Cupule acétabulaire artificielle pouvant être vissée (34) présentant n'importe quel contour extérieur d'enveloppe de coque, par exemple sphérique, parasphérique, conique, conique-sphérique, parabolique, avec un profil se rétrécissant en direction de la tête de dent de la dent filetée, par exemple sous forme de filetage en pointe, n'importe quelle position de dent, par exemple neutre ou basculée vers le pôle de la cupule, et n'importe quelle pente, par exemple une pente constante ou variable,
**caractérisée en ce que**
les ailes filetées (35, 36, 40) ou nervures filetées individuelles séparées les unes des autres par des rainures de serrage (44) sont constituées par des surfaces de vissage de diamètre constant, les ailes filetées suivantes dans le sens de vissage (36) présentant des diamètres supérieurs à ceux des précédentes (35).

2. Cupule acétabulaire artificielle selon la revendication 1, **caractérisée en ce que** les ailes filetées (35, 36, 40) sont, dans leur extension, pivotées suivant un petit angle les unes par rapport aux autres en direction de l'angle de torsion des rainures de serrage (44).
